# EUROPEAN PATENT APPLICATION

(11) **EP 3 854 395 A1**
(43) Date of publication of application: **28.07.2021**
(21) Application number: 19863028.7
(22) Date of filing: 18.09.2019
(51) Int. Cl.: A61K 31/47, A61K 31/58, A61P 13/08, A61P 19/00, A61P 35/00, A61P 35/04, A61P 43/00

(54) **COMBINATION THERAPY OF ACYLTHIOUREA COMPOUND AND ABIRATERONE**

(30) Priority: 18.09.2018 JP 2018174221
(71) Applicant: Taiho Pharmaceutical Co., Ltd., Chiyoda-ku Tokyo 101-8444 (JP)
(72) Inventor: ISHIBASHI, Yuko, Tokyo 101-0047 (JP)
(74) Representative: Blodig, Wolfgang
(86) International application number: PCT/JP2019/036530
(87) International publication number: WO 2020/059744

(57) **Abstract**

Combination therapy of cancer using a c-Met selective inhibitor, an acylthiourea compound or a pharmaceutically acceptable salt thereof, and showing an excellent antitumor effect with less side effects is provided. An antitumor agent contains a compound represented by the following formula (I) or a pharmaceutically acceptable salt thereof: 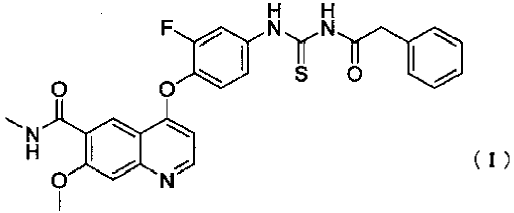 characterized by being used in combination with abiraterone or a derivative thereof.

## Description

### Field of the Invention

The present invention relates to combination therapy of cancer using an acylthiourea compound or a pharmaceutically acceptable salt thereof.

### Background of the Invention

Abiraterone acetate is a compound that is rapidly hydrolyzed to abiraterone in vivo and irreversibly and selectively inhibits the activity of 17α-hydroxylase/C17,20-lyase (CYP17) which is an androgen-synthesizing enzyme. Abiraterone is reported to show an antitumor effect against castration-resistant prostate cancer through inhibition of androgen synthesis in, in particular, the testis, adrenal gland, and tumor tissue (Non Patent Literatures 1 and 2) and is further reported to show clinical efficacy also against prostate cancer having a high-risk prognostic factor without endocrine therapy treatment (Non Patent Literature 3), and the adaptation diseases thereof are castration-resistant prostate cancer and prostate cancer having a high-risk prognostic factor without endocrine therapy treatment.

On the other hand, an acylthiourea compound having a specific structure or a pharmaceutically acceptable salt thereof was found to have c-Met inhibitory action by the group of the present inventors (Patent Literature 1) and was reported to be effective for bone metastasis of lung cancer (Non Patent Literature 4). Furthermore, an antitumor effect by combination with various antitumor agents has been reported (Patent Literature 2). In addition, it has been reported that an acylthiourea compound or a pharmaceutically acceptable salt thereof is also effective against osteoporosis (Patent Literature 3) and fibrosis (Patent Literature 4). Furthermore, crystals of the acylthiourea compound or a pharmaceutically acceptable salt thereof (Patent Literature 5) and a pharmaceutical composition including an acylthiourea compound or a pharmaceutically acceptable salt thereof and hydroxypropyl-β-cyclodextrin (Patent Literature 6) have been reported.

In addition, recently, clinical trials of the acylthiourea compound or a pharmaceutically acceptable salt thereof has been conducted in patients with castration-resistant prostate cancer (Non Patent Literatures 5 and 6). In addition, the effect of the combination use of cabozantinib having c-Met inhibitory action and abiraterone in the same cancer has been reported (Patent Literature 7) .

### Citation List

### Patent Literature

[Patent Literature 1] International Publication No. WO 2009/125597
[Patent Literature 2] International Publication No. WO 2013/100014
[Patent Literature 3] International Publication No. WO 2015/046484
[Patent Literature 4] International Publication No. WO 2016/208744
[Patent Literature 5] International Publication No. WO 2016/175305
[Patent Literature 6] International Publication No. WO 2018/151177
[Patent Literature 7] International Publication No. WO 2014/165779

### Non Patent Literature

[Non Patent Literature 1] N. Engl. J. Med., 2011, 364: 1995-2005
[Non Patent Literature 2] N. Engl. J. Med., 2013, 368: 138-48
[Non Patent Literature 3] Lancet Oncol., 2018, 19: 194-206
[Non Patent Literature 4] H. Fujita, et al., PLoS ONE, volume 11(10), e0164830, 2016
[Non Patent Literature 5] N. Matsubara, et al., Annals of Oncology, Volume 28, Issue suppl. 5, 1 September, 2017
[Non Patent Literature 6] https://rctportal.niph.go.jp/s/detail/jp?trial_id=JapicCTI-163448

### Summary of the Invention

### Problems to be Solved by the Invention

It is an object of the present invention to provide combination therapy of cancer using a c-Met selective inhibitor, an acylthiourea compound or a pharmaceutically acceptable salt thereof, and showing an excellent antitumor effect with less side effects.

### Means for Solving the Problems

The present inventor has found that antitumor therapy by combinational administration of an acylthiourea compound having a specific structure or a pharmaceutically acceptable salt thereof and abiraterone or a derivative thereof exhibits excellent antitumor effect and antitumor enhancing effect without reinforcing side-effects, and the present invention has been accomplished.

The present invention provides the following [1] to [16]:
[1] An antitumor agent containing a compound represented by the following formula (I) or a pharmaceutically acceptable salt thereof, being administered in combination with abiraterone or a derivative thereof;
[2] The antitumor agent according to aspect [1], wherein the pharmaceutically acceptable salt is a methanesulfonate salt;
[3] The antitumor agent according to aspect [1] or [2], wherein the abiraterone or a derivative thereof is abiraterone acetate;
[4] The antitumor agent according to aspects [1] to [3], being administered to a human;
[5] The antitumor agent according to any one of aspects [1] to [4] for treating castration-resistant prostate cancer;
[6] The antitumor agent according to any one of aspects [1] to [5] for treating castration-resistant prostate cancer with bone metastasis;
[7] The antitumor agent according to any one of aspects [1] to [6], wherein the compound represented by formula (I) or a pharmaceutically acceptable salt thereof is administered once a day in an amount of from 200 mg to 400 mg as the free form of the compound I;
[8] The antitumor agent according to any one of aspects [1] to [6], wherein the compound represented by formula (I) or a pharmaceutically acceptable salt thereof is administered once a day in an amount of 300 mg as the free form of the compound I;
[9] The antitumor agent according to any one of aspects [1] to [8], wherein the compound represented by formula (I) or a pharmaceutically acceptable salt thereof is administered for 5 days, and a drug holiday for 2 days is then taken;
[10] An antitumor effect-enhancing agent for abiraterone or a derivative thereof, comprising a compound represented by the following formula (I) or a pharmaceutically acceptable salt thereof as an active ingredient;
[11] A method for treating a tumor, comprising administering therapeutically effective amounts of a compound represented by the following formula (I) or a pharmaceutically acceptable salt thereof and abiraterone or a derivative thereof to a patient;
[12] A compound represented by the following formula (I) or a pharmaceutically acceptable salt thereof for use in combination with abiraterone or a derivative thereof in the treatment of a tumor;
[13] Use of a compound represented by the following formula (I) or a pharmaceutically acceptable salt thereof for manufacturing an antitumor agent to be administered in combination with abiraterone or a derivative thereof;
[14] A method for enhancing an antitumor effect of abiraterone or a derivative thereof, comprising administering a therapeutically effective amount of a compound represented by the following formula (I) or a pharmaceutically acceptable salt thereof to a patient;
[15] A compound represented by the following formula (I) or a pharmaceutically acceptable salt thereof for enhancing an antitumor effect of abiraterone or a derivative thereof; and
[16] Use of a compound represented by the following formula (I) or a pharmaceutically acceptable salt thereof for manufacturing an antitumor effect-enhancing agent for enhancing an antitumor effect of abiraterone or a derivative thereof.

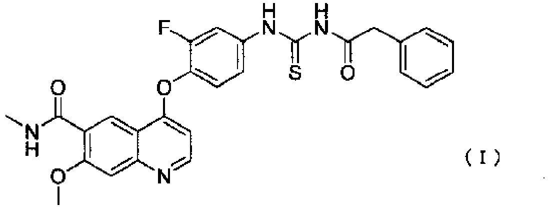

### Brief Description of Drawings

[Figure 1] Figure 1 shows changes in the BSI value at the best response in each dosage group of compound I used in combination with abiraterone. The vertical axis shows the BSI when the highest effect was exhibited among BSI measured during administration of compound I and within 3 months after discontinuation of the administration based on the bone metastasis in the test before the start of the administration in each dosage group.
[Figure 2] Figure 2 shows the administration period of each dosage group of compound I used in combination with abiraterone. The horizontal axis shows the number of days of the administration period for all cases (n = 24).
[Figure 3] Figure 3 shows the administration period of each dosage group of compound I used in combination with abiraterone. The horizontal axis shows the number of days of the administration period for all cases (n = 24).

### Detailed Description of the Invention

The acylthiourea compound for use in the present invention is 4-(2-fluoro-4-(3-(2-phenylacetyl)thioureido)phenoxy)-7-methoxy-N-methylquinoline-6-carboxamide (hereinafter, referred to as compound I in the present specification) represented by the following formula (I): 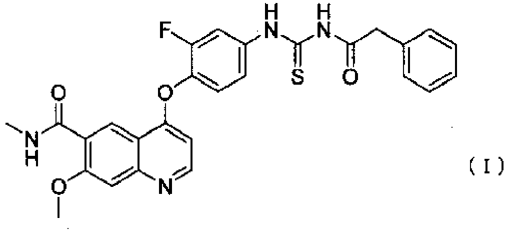 and is a known compound as a c-Met inhibitor.

The compound I is one of acylthiourea compounds described in International Publication No. WO 2009/125597 and International Publication No. WO 2016/175305 and can be synthesized in accordance with the methods described therein.

The compound I is obtained as a white solid and can be present as a crystal. The compound I can be administered as a free form or a pharmaceutically acceptable salt.

Examples of the pharmaceutically acceptable salt of the compound I include salts with inorganic acids, such as hydrochloric acid, hydrobromic acid, hydroiodic acid, sulfuric acid, nitric acid, and phosphoric acid, and organic acids, such as formic acid, acetic acid, propionic acid, oxalic acid, malonic acid, succinic acid, ascorbic acid, isoascorbic acid, mandelic acid, fumaric acid, aspartic acid, maleic acid, lactic acid, malic acid, hippuric acid, glutaric acid, adipic acid, citric acid, tartaric acid, carbonic acid, picric acid, methanesulfonic acid (mesylic acid), p-toluenesulfonic acid (p-tosyl acid), and glutamic acid; and salts with alkali metals, alkaline earth metals, and other bases, such as a potassium salt, a sodium salt, a calcium salt, an ammonium salt, a methylammonium salt, and a dimethylammonium salt.

Among these salts, an example of the salt that can be suitably used is a methanesulfonate salt, and more preferably, the methanesulfonate salt may be a salt of methanesulfonic acid and compound I at a molar ratio of 1:1. The methanesulfonate salt can be in the form of a single crystal, a mixture of two or more crystal polymorphisms, amorphous, or a mixture thereof.

In addition, abiraterone of which chemical name is 17-(pyridin-3-yl)androsta-5,16-dien-3β-ol has the following structure:

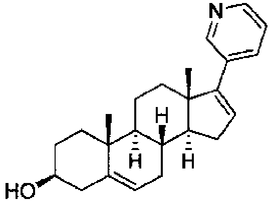

Abiraterone has CYP17A1 inhibitory action, and a metabolic precursor thereof, an acetate ester represented by the following formula (17-(pyridin-3-yl)androsta-5,16-dien-3β-yl acetate: abiraterone acetate), is being marketed as a prostate cancer therapeutic agent (trade name: Zytiga).

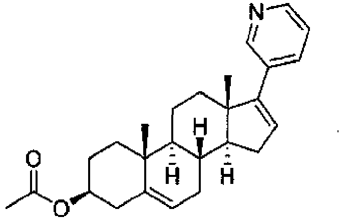

In the present invention, examples of the abiraterone or a derivative thereof that can be administered in combination with a compound I or a pharmaceutically acceptable salt thereof include, in addition to abiraterone, metabolic precursors of abiraterone as abiraterone derivatives, i.e., esters of various acids, such as abiraterone acetate, abiraterone formate, abiraterone propionate, abiraterone butanoate, abiraterone 2-methylpropionate, abiraterone phosphate, abiraterone sulfate, abiraterone methanesulfonate, glycine abiraterone, and alanine abiraterone; various alkyl ethers, such as methyl abiraterone (3-[(3β)-3-methoxyandrosta-5,16-dien-17-yl]pyridine), methoxymethyl abiraterone, benzyl abiraterone, tetrahydropyran abiraterone, and trityl abiraterone; various carbonates, such as ethoxycarbonyl abiraterone and propoxycarbonyl; and various carbamates, such as methylaminocarbonyl abiraterone. Preferably, the derivative is abiraterone acetate.

As shown in Examples described below, a methanesulfonate salt of compound I exhibits an excellent effect of suppressing bone metastasis without causing serious side effects when administered in combination with abiraterone acetate to a castration-resistant prostate cancer patient.

Accordingly, the compound I or a pharmaceutically acceptable salt thereof can be used, in the case of being administered in combination with abiraterone or a derivative thereof, as an antitumor agent exhibiting an excellent antitumor effect, preferably a suppressing effect on tumor recurrence or metastasis, more preferably as an antitumor agent for preventing or treating castration-resistant prostate cancer associated with bone metastasis, or as an inhibitor of castration-resistant prostate cancer metastasis to bone.

In addition, the compound I or a pharmaceutically acceptable salt thereof can be used as an antitumor effect-enhancing agent that enhances the antitumor effect of abiraterone or a derivative thereof, in particular, the antitumor effect on castration-resistant prostate cancer associated with bone metastasis. Furthermore, abiraterone or a derivative thereof can be used as an antitumor effect-enhancing agent that enhances the antitumor effect of the compound I or a pharmaceutically acceptable salt thereof, in particular, the antitumor effect against castration-resistant prostate cancer associated with bone metastasis.

In the antitumor agent or antitumor effect-enhancing agent of the present invention, the malignant tumor that is a target of the treatment and the antitumor effect enhancement is, for example, epithelial cancer (respiratory system cancer, digestive system cancer, reproductive system cancer, secretory system cancer, etc.), sarcoma, hematopoietic cell tumor (B-cell lymphoma, chronic lymphocytic leukemia, peripheral T-cell lymphoma, myelodysplastic syndrome, acute myeloid leukemia, acute lymphocytic leukemia, etc.), central nervous system tumor, peripheral nervous system tumor, or multiple myeloma. The type of cancer as the target is preferably epithelial cancer and more preferably reproductive system cancer.

In addition, the type of organ that develops a tumor is not particularly limited, and examples of the tumor include head and neck cancer such as parotid gland cancer, esophageal cancer, stomach cancer, duodenal cancer, colon cancer, rectal cancer, liver cancer, gallbladder/bile duct cancer, biliary tract cancer, pancreatic cancer, lung cancer, breast cancer, ovarian cancer, fallopian tube cancer, cervical cancer, Endometrial cancer, renal cancer, adrenal cancer, bladder cancer, prostate cancer, urothelial cancer, testicular tumor, bone/soft tissue sarcoma, hematological cancer, multiple myeloma, skin cancer, brain tumor, and mesothelioma. The tumor that is a target of treatment is preferably prostate cancer and more preferably castration-resistant prostate cancer.

The antitumor action of the antitumor agent of the present invention encompasses inhibition of tumor growth, delay in tumor growth, tumor regression, and tumor contraction, and also suppression of tumor recurrence and metastasis.

The metastatic site of cancer differs depending on the primary lesion and the characteristics of the cancer. As the primary cancer in the present invention, among the above-mentioned cancers, prostate cancer, more preferably castration-resistant prostate cancer, is mentioned. In addition, in such a case, the site of metastasis from the primary region is mainly bone, and the antitumor agent of the present invention is particularly useful for suppressing bone metastasis of castration-resistant prostate cancer. In the treatment of castration-resistant prostate cancer, prostate cancer itself, which is the primary lesion, may be removed by castration, or a castrated state may be maintained by an agent. In such a case, the antitumor effect against castration-resistant prostate cancer is evaluated by the antitumor effect at the site of metastasis.

Accordingly, the present invention relates to a tumor metastasis inhibitor comprising the compound I or a pharmaceutically acceptable salt characterized by being used in combination with an abiraterone derivative, preferably an inhibitor of tumor metastasis to bone, more preferably an inhibitor of prostate cancer metastasis to bone, and more preferably an inhibitor of castration-resistant prostate cancer metastasis to bone.

In addition, the antitumor effect-enhancing agent according to the present invention enhances, when one agent has an antitumor effect, the antitumor action of the other agent.

When a compound represented by formula (I) or a pharmaceutically acceptable salt thereof of the present invention is administered in combination with abiraterone or a derivative thereof, another antitumor agent may be further administered. Such additional antitumor agent is not particularly limited as long as it has antitumor action.

The compound I or a pharmaceutically acceptable salt thereof is provided as a preparation (pharmaceutical composition) comprising them as active ingredients and further appropriately blended with a pharmaceutical carrier. As the pharmaceutical carrier that is used here, various organic or inorganic carrier materials that are commonly used as preparation materials are used. For example, an excipient agent, a binder, a disintegrant, a lubricant, and a colorant can be blended in a solid preparation. In addition, a dissolution aid, a suspending agent, an isotonic agent, a buffering agent, and a soothing agent can be blended in a liquid preparation. As needed, formulation additives, such as a preservative, an antioxidant, a sweetener, and a stabilizer, can be used.

When an oral solid preparation is prepared, first, to the compound I or a pharmaceutically acceptable salt thereof, an excipient agent and as needed, for example, another excipient agent, a binder, a disintegrant, a lubricant, a colorant, and a corrigent agent are added. Subsequently, tablets, coated tablets, granules, powders, and capsules can be manufactured by usual methods. When an injection is prepared, first, to the compound, a pH adjuster, a buffering agent, a stabilizer, an isotonic agent, a local anesthetic, etc. are added. Subsequently, subcutaneous, intramuscular, and intravenous injections can be manufactured by usual methods. The compound I or a pharmaceutically acceptable salt thereof is preferably orally administered because it has excellent oral absorbability.

In the present invention, abiraterone or a derivative thereof that is administered in combination with the compound I or a pharmaceutically acceptable salt thereof depends on the type of each agent and is administered by, for example, intravenous administration, intraperitoneal administration, suppository administration, or oral administration, preferably oral administration.

In addition, abiraterone or a derivative thereof and the compound I or a pharmaceutically acceptable salt thereof can be provided separately in respective preparations or provided as a compounding agent. Furthermore, abiraterone or a derivative thereof and the compound I or a pharmaceutically acceptable salt thereof can be provided as a kit preparation including them as separate preparations composed of them alone.

In the present invention, the dose of the compound I or a pharmaceutically acceptable salt thereof is not particularly limited and can be appropriately set based on the cancer, side effects, etc. The dose may be, as the free form of the compound I, 25 mg or more, preferably from 50 mg to 1000 mg, more preferably from 150 mg to 450 mg, further preferably from 200 mg to 400 mg, even more preferably from 250 mg to 350 mg, and particularly preferably 300 mg.

In addition, in another embodiment, the dose of the compound I or a pharmaceutically acceptable salt thereof is, as the free form of the compound I, one selected from the group consisting of 100 mg, 200 mg, 250 mg, 300 mg, 350 mg, 400 mg, 450 mg, 500 mg, 550 mg, 600 mg, 650 mg, and 700 mg, preferably one selected from the group consisting of 200 mg, 250 mg, 300 mg, 350 mg, 400 mg, 450 mg, and 500 mg, and more preferably one selected from the group consisting of 200 mg, 300 mg, and 400 mg.

In addition, in the present invention, the compound I or a pharmaceutically acceptable salt thereof is used in a dose of from 0.1 to 1.0 times the recommended dose (RD) when the compound I or a pharmaceutically acceptable salt thereof is administered alone and is preferably used in a dose of from 0.3 to 0.7 times, more preferably from 0.4 to 0.7 times, and more preferably from 0.4 to 0.5 times the RD.

In the present invention, the compound I or a pharmaceutically acceptable salt thereof can be administered once a day.

In addition, in the present invention, the compound I or a pharmaceutically acceptable salt thereof may be administered every day, or a drug holiday period may be provided based on the cancer type, side effects, etc. as appropriate. Preferably, the compound I or a pharmaceutically acceptable salt thereof is administered for 5 days, and a drug holiday for 2 days is taken.

In the present invention, the dose of abiraterone or a derivative thereof is not particularly limited and can be appropriately set based on the cancer type, side effects, etc. The dose may be 200 mg or more, preferably from 400 mg to 1600 mg, more preferably from 700 mg to 1300 mg, and particularly preferably from 900 mg to 1100 mg.

In addition, when abiraterone or a derivative thereof is abiraterone acetate, the same dose as above is set. In addition, in another embodiment, the dose of abiraterone acetate may be one selected from the group consisting of 250 mg, 500 mg, 750 mg, 1000 mg, 1250 mg, and 1500 mg, preferably one selected from the group consisting of 250 mg, 500 mg, 750 mg, and 1000 mg, more preferably one selected from the group consisting of 500 mg, 750 mg, and 1000 mg, more preferably one selected from the group consisting of 750 mg and 1000 mg, and particularly preferably 1000 mg.

In the present invention, abiraterone or a derivative thereof can be administered once or twice or more times a day. The number of administration per day may be from 1 to 3 and is preferably once.

In addition, in the present invention, abiraterone or a derivative thereof may be administered every day, or a drug holiday period may be provided based on the cancer type, side effects, etc. as appropriate. Preferably, abiraterone or a derivative thereof is administered every day.

The administration molar ratio of the compound I or a pharmaceutically acceptable salt thereof per day may be from 0.1 to 1.0 as the moles of the free form of the compound I with respect to 1 mole of abiraterone or a derivative thereof and is preferably from 0.15 to 0.30 moles, more preferably from 0.20 to 0.25 moles, and more preferably 0.23 moles.

In the present invention, numerical values mentioned about, for example, doses of abiraterone or a derivative thereof and the compound I or a pharmaceutically acceptable salt thereof and ratio between these agents are intended to include values within the range of ±20% or ±10% variation from the stated values. Those skilled in the art will recognize that doses or ratios within such ranges can produce effects similar to those when the doses or ratios are those mentioned above.

The animal species to which compound I or a pharmaceutically acceptable salt thereof can be administered may be a mammal and is preferably, in addition to a mammal as a target of treatment, a mammal that is generally used in animal experiments of pharmacokinetics, pharmacological effectiveness test, etc. or a human to be administered in a clinical trial, specifically, a human, a rat, a mouse, a rabbit, and a dog, and preferably a human.

In the present invention, regardless of the treatment history of cancer before the start of administration of compound I or a pharmaceutically acceptable salt thereof and abiraterone or a derivative thereof, the antitumor effect and treatment or prevention of tumor metastasis can be achieved. The treatment history may be that established as a standard treatment or may be treatment according to individual symptoms. It is known that the system of standard treatment varies depending on the cancer.

The standard pharmacotherapy of prostate cancer is endocrine therapy (hormone therapy), and the endocrine therapy first received after prostate cancer affection is called primary endocrine therapy. When any of recurrence of prostate-specific antigen (PSA), exacerbation of metastatic lesion, and appearance of new lesion is observed after primary endocrine therapy, it is diagnosed as castration-resistant prostate cancer. The standard treatment for this case is administration of docetaxel, abiraterone acetate, enzalutamide (trade name: XTANDI), cabazitaxel (trade name: JEVTANA), radium-223 (trade name: Xofigo), or the like. The present invention can also be applied to castration-resistant prostate cancer before and after treatment with docetaxel, regardless of whether treatment with enzalutamide or radium-223 is performed or not.

As described above, the present invention relates to an antitumor agent including the compound I or a pharmaceutically acceptable salt thereof characterized by being administered in combination with abiraterone or a derivative thereof and preferably an antitumor agent including the compound I or a pharmaceutically acceptable salt thereof characterized by being administered in combination with abiraterone acetate.

More preferably, the invention relates to an antitumor agent including the compound I or a pharmaceutically acceptable salt thereof characterized by being administered to a human in combination with abiraterone acetate.

More preferably, the invention relates to an antitumor agent for treating castration-resistant prostate cancer, including the compound I or a pharmaceutically acceptable salt thereof characterized by being administered to a human in combination with abiraterone acetate.

More preferably, the invention relates to an antitumor agent for treating castration-resistant prostate cancer, including the compound I or a pharmaceutically acceptable salt thereof in a dose of from 200 mg to 400 mg as the free form of the compound I characterized by being administered to a human in combination with abiraterone acetate in a dose of from 250 mg to 1000 mg.

More preferably, the invention relates to an antitumor agent for treating castration-resistant prostate cancer with bone metastasis, including the compound I or a pharmaceutically acceptable salt thereof in a dose of from 200 mg to 400 mg as the free form of the compound I characterized by being administered to a human in combination with abiraterone acetate in a dose of from 250 mg to 1000 mg.

More preferably, the invention relates to an antitumor agent for treating castration-resistant prostate cancer with bone metastasis, including the compound I or a pharmaceutically acceptable salt thereof to be administered once a day in an amount of from 200 mg to 400 mg as the free form of the compound I, characterized by being administered to a human in combination with from 250 mg to 1000 mg of abiraterone acetate to be administered once a day.

More preferably, the invention relates to an antitumor agent for treating castration-resistant prostate cancer with bone metastasis, including the compound I or a pharmaceutically acceptable salt thereof to be administered once a day in a dose selected from the group consisting of 200 mg, 300 mg, and 400 mg as the free form of the compound I, characterized by being administered to a human in combination with abiraterone acetate to be administered once a day in a dose selected from the group consisting of 250 mg, 500 mg, 750 mg, and 1000 mg.

More preferably, the invention relates to an antitumor agent for treating castration-resistant prostate cancer with bone metastasis, including the compound I or a pharmaceutically acceptable salt thereof to be administered once a day in a dose selected from the group consisting of 200 mg, 300 mg, and 400 mg as the free form of the compound I, characterized by being administered to a human in combination with abiraterone acetate to be administered once a day in a dose selected from the group consisting of 500 mg, 750 mg, and 1000 mg.

More preferably, the invention relates to an antitumor agent for treating castration-resistant prostate cancer with bone metastasis, including the compound I or a pharmaceutically acceptable salt thereof to be administered once a day in a dose selected from the group consisting of 200 mg, 300 mg, and 400 mg as the free form of the compound I, characterized by being administered to a human in combination with abiraterone acetate to be administered once a day in a dose selected from the group consisting of 750 mg and 1000 mg.

More preferably, the invention relates to an antitumor agent for treating castration-resistant prostate cancer with bone metastasis, including the compound I or a pharmaceutically acceptable salt thereof to be administered once a day in a dose selected from the group consisting of 200 mg and 300 mg as the free form of the compound I, characterized by being administered to a human in combination with abiraterone acetate to be administered once a day in a dose of 1000 mg.

More preferably, the invention relates to an antitumor agent for treating castration-resistant prostate cancer with bone metastasis, including the compound I or a pharmaceutically acceptable salt thereof to be administered once a day in an amount of 300 mg as the free form of the compound I, characterized by being administered to a human in combination with 1000 mg of abiraterone acetate to be administered once a day.

More preferably, the invention relates to an antitumor agent for treating castration-resistant prostate cancer with bone metastasis, including the compound I or a pharmaceutically acceptable salt thereof to be administered to a human once a day in an amount of 300 mg as the free form of the compound I for from 3 days to 5 days and then taking a drug holiday for 1 to 3 days, characterized by being administered in combination with 1000 mg of abiraterone acetate to be administered once a day.

More preferably, the invention relates to an antitumor agent for treating castration-resistant prostate cancer with bone metastasis, including the compound I or a pharmaceutically acceptable salt thereof to be administered to a human once a day in an amount of 300 mg as the free form of the compound I for 5 days and then taking a drug holiday for 2 days, characterized by being administered in combination with 1000 mg of abiraterone acetate to be administered once a day.

In addition, the present invention relates to an antitumor effect-enhancing agent for abiraterone or a derivative thereof, including the compound I or a pharmaceutically acceptable salt thereof as an active ingredient.

More preferably, the invention relates to an antitumor effect-enhancing agent for abiraterone acetate, including the compound I or a pharmaceutically acceptable salt thereof as an active ingredient.

More preferably, the invention relates to an antitumor effect-enhancing agent for abiraterone acetate, including the compound I or a pharmaceutically acceptable salt thereof as an active ingredient and being administered to a human.

More preferably, the invention relates to an antitumor effect-enhancing agent for abiraterone acetate for treating castration-resistant prostate cancer, including the compound I or a pharmaceutically acceptable salt thereof as an active ingredient and being administered to a human.

More preferably, the invention relates to an antitumor effect-enhancing agent for abiraterone acetate for treating castration-resistant prostate cancer to be administered in a dose of from 250 mg to 1000 mg, including the compound I or a pharmaceutically acceptable salt thereof as an active ingredient to be administered to a human in a dose of from 200 mg to 400 mg as the free form of the compound I.

More preferably, the invention relates to an antitumor effect-enhancing agent for abiraterone acetate for treating castration-resistant prostate cancer with bone metastasis to be administered in a dose of from 250 mg to 1000 mg, including the compound I or a pharmaceutically acceptable salt thereof as an active ingredient to be administered to a human in a dose of from 200 mg to 400 mg as the free form of the compound I.

More preferably, the invention relates to an antitumor effect-enhancing agent for abiraterone acetate for treating castration-resistant prostate cancer with bone metastasis to be administered once a day in an amount of from 250 mg to 1000 mg, including the compound I or a pharmaceutically acceptable salt thereof as an active ingredient to be administered to a human once a day in an amount of from 200 mg to 400 mg as the free form of the compound I.

More preferably, the invention relates to an antitumor effect-enhancing agent for abiraterone acetate for treating castration-resistant prostate cancer with bone metastasis to be administered once a day in a dose selected from the group consisting of 250 mg, 500 mg, 750 mg, and 1000 mg, including the compound I or a pharmaceutically acceptable salt thereof as an active ingredient to be administered to a human once a day in a dose selected from the group consisting of 200 mg, 300 mg, and 400 mg as the free form of the compound I.

More preferably, the invention relates to an antitumor effect-enhancing agent for abiraterone acetate for treating castration-resistant prostate cancer with bone metastasis to be administered once a day in a dose selected from the group consisting of 750 mg and 1000 mg, including the compound I or a pharmaceutically acceptable salt thereof as an active ingredient to be administered to a human once a day in a dose selected from the group consisting of 200 mg, 300 mg, and 400 mg as the free form of the compound I.

More preferably, the invention relates to an antitumor effect-enhancing agent for abiraterone acetate for treating castration-resistant prostate cancer with bone metastasis to be administered once a day in a dose selected from the group consisting of 500 mg, 750 mg, and 1000 mg, including the compound I or a pharmaceutically acceptable salt thereof as an active ingredient to be administered to a human once a day in a dose selected from the group consisting of 200 mg, 300 mg, and 400 mg as the free form of the compound I.

More preferably, the invention relates to an antitumor effect-enhancing agent for abiraterone acetate for treating castration-resistant prostate cancer with bone metastasis to be administered once a day in a dose selected from the group consisting of 750 mg and 1000 mg, including the compound I or a pharmaceutically acceptable salt thereof as an active ingredient to be administered to a human once a day in a dose selected from the group consisting of 200 mg, 300 mg, and 400 mg as the free form of the compound I.

More preferably, the invention relates to an antitumor effect-enhancing agent for abiraterone acetate for treating castration-resistant prostate cancer with bone metastasis to be administered once a day in a dose selected from the group consisting of 750 mg and 1000 mg, including the compound I or a pharmaceutically acceptable salt thereof as an active ingredient to be administered to a human once a day in a dose selected from the group consisting of 200 mg and 300 mg as the free form of the compound I.

More preferably, the invention relates to an antitumor effect-enhancing agent for abiraterone acetate for treating castration-resistant prostate cancer with bone metastasis to be administered once a day in an amount of 1000 mg, including the compound I or a pharmaceutically acceptable salt thereof as an active ingredient to be administered to a human once a day in a dose selected from the group consisting of 200 mg and 300 mg as the free form of the compound I.

More preferably, the invention relates to an antitumor effect-enhancing agent for abiraterone acetate for treating castration-resistant prostate cancer with bone metastasis to be administered once a day in an amount of 1000 mg, including the compound I or a pharmaceutically acceptable salt thereof as an active ingredient to be administered to a human once a day in an amount of 300 mg as the free form of the compound I.

More preferably, the invention relates to an antitumor effect-enhancing agent for abiraterone acetate for treating castration-resistant prostate cancer with bone metastasis to be administered once a day in an amount of 1000 mg, including the compound I or a pharmaceutically acceptable salt thereof as an active ingredient to be administered to a human once a day in an amount of 300 mg as the free form of the compound I for 5 days and then taking a drug holiday for 2 days.

In addition, the present invention relates to a method for treating a tumor, comprising administering therapeutically effective amounts of the compound I or a pharmaceutically acceptable salt thereof and abiraterone or a derivative thereof to a patient.

More preferably, the invention relates to a method for treating a human tumor, comprising administering therapeutically effective amounts of the compound I or a pharmaceutically acceptable salt thereof and abiraterone acetate to a patient.

More preferably, the invention relates to a method for treating human castration-resistant prostate cancer, comprising administering therapeutically effective amounts of the compound I or a pharmaceutically acceptable salt thereof and abiraterone acetate to a patient.

More preferably, the invention relates to a method for treating human castration-resistant prostate cancer, comprising administering therapeutically effective amounts of the compound I or a pharmaceutically acceptable salt thereof in a dose of from 200 mg to 400 mg as the free form of the compound I and abiraterone acetate in a dose of from 250 mg to 1000 mg to a patient.

More preferably, the invention relates to a method for treating human castration-resistant prostate cancer with bone metastasis, comprising administering therapeutically effective amounts of the compound I or a pharmaceutically acceptable salt thereof in a dose of from 200 mg to 400 mg as the free form of the compound I and abiraterone acetate in a dose of from 250 mg to 1000 mg to a patient.

More preferably, the invention relates to a method for treating human castration-resistant prostate cancer with bone metastasis, comprising administering therapeutically effective amounts of the compound I or a pharmaceutically acceptable salt thereof in an amount of from 200 mg to 400 mg as the free form of the compound I once a day and abiraterone acetate in an amount of from 250 mg to 1000 mg once a day to a patient.

More preferably, the invention relates to a method for treating human castration-resistant prostate cancer with bone metastasis, comprising administering therapeutically effective amounts of the compound I or a pharmaceutically acceptable salt thereof in a dose selected from the group consisting of 200 mg, 300 mg, and 400 mg as the free form of the compound I once a day and abiraterone acetate in a dose selected from the group consisting of 250 mg, 500 mg, 750 mg, and 1000 mg once a day to a patient.

More preferably, the invention relates to a method for treating human castration-resistant prostate cancer with bone metastasis, comprising administering therapeutically effective amounts of the compound I or a pharmaceutically acceptable salt thereof in a dose selected from the group consisting of 200 mg, 300 mg, and 400 mg as the free form of the compound I once a day and abiraterone acetate in a dose selected from the group consisting of 250 mg, 500 mg, 750 mg, and 1000 mg once a day to a patient.

More preferably, the invention relates to a method for treating human castration-resistant prostate cancer with bone metastasis, comprising administering therapeutically effective amounts of the compound I or a pharmaceutically acceptable salt thereof in a dose selected from the group consisting of 200 mg, 300 mg, and 400 mg as the free form of the compound I once a day and abiraterone acetate in a dose selected from the group consisting of 500 mg, 750 mg, and 1000 mg once a day to a patient.

More preferably, the invention relates to a method for treating human castration-resistant prostate cancer with bone metastasis, comprising administering therapeutically effective amounts of the compound I or a pharmaceutically acceptable salt thereof in a dose selected from the group consisting of 200 mg, 300 mg, and 400 mg as the free form of the compound I once a day and abiraterone acetate in a dose selected from the group consisting of 750 mg and 1000 mg once a day to a patient.

More preferably, the invention relates to a method for treating human castration-resistant prostate cancer with bone metastasis, comprising administering therapeutically effective amounts of the compound I or a pharmaceutically acceptable salt thereof in a dose selected from the group consisting of 200 mg, 300 mg, and 400 mg as the free form of the compound I once a day and 1000 mg of abiraterone acetate once a day to a patient.

More preferably, the invention relates to a method for treating human castration-resistant prostate cancer with bone metastasis, comprising administering therapeutically effective amounts of the compound I or a pharmaceutically acceptable salt thereof in a dose selected from the group consisting of 200 mg and 300 mg once a day and 1000 mg of abiraterone acetate once a day to a patient.

More preferably, the invention relates to a method for treating human castration-resistant prostate cancer with bone metastasis, comprising administering therapeutically effective amounts of the compound I or a pharmaceutically acceptable salt thereof once a day in an amount of 300 mg as the free form of the compound I for from 3 days to 5 days and then taking a drug holiday for from 1 day to 3 days and 1000 mg of abiraterone acetate once a day to a patient.

More preferably, the invention relates to a method for treating human castration-resistant prostate cancer with bone metastasis, comprising administering therapeutically effective amounts of the compound I or a pharmaceutically acceptable salt thereof once a day in an amount of 300 mg as the free form of the compound I for 5 days and then taking a drug holiday for 2 days and 1000 mg of abiraterone acetate once a day to a patient.

In addition, the present invention relates to the compound I or a pharmaceutically acceptable salt thereof for use in combination with abiraterone or a derivative thereof in the treatment of a tumor.

More preferably, the invention relates to the compound I or a pharmaceutically acceptable salt thereof for use in combination with abiraterone acetate in the treatment of a tumor.

More preferably, the invention relates to the compound I or a pharmaceutically acceptable salt thereof for use in combination with abiraterone acetate in the treatment of a tumor by being administered to a human.

More preferably, the invention relates to the compound I or a pharmaceutically acceptable salt thereof for use in combination with abiraterone acetate in the treatment of castration-resistant prostate cancer by being administered to a human.

More preferably, the invention relates to the compound I or a pharmaceutically acceptable salt thereof for use in a dose of from 200 mg to 400 mg as the free form of the compound I in combination with abiraterone acetate in a dose of from 250 mg to 1000 mg in the treatment of castration-resistant prostate cancer by being administered to a human.

More preferably, the invention relates to the compound I or a pharmaceutically acceptable salt thereof for use in a dose of from 200 mg to 400 mg as the free form of the compound I in combination with abiraterone acetate in a dose of from 250 mg to 1000 mg in the treatment of castration-resistant prostate cancer with bone metastasis by being administered to a human.

More preferably, the invention relates to the compound I or a pharmaceutically acceptable salt thereof to be administered once a day in an amount of from 200 mg to 400 mg as the free form of the compound I for use in combination with abiraterone acetate to be administered once a day in a dose of from 250 mg to 1000 mg in the treatment of castration-resistant prostate cancer with bone metastasis by being administered to a human.

More preferably, the invention relates to the compound I or a pharmaceutically acceptable salt thereof to be administered once a day in a dose selected from the group consisting of 200 mg, 300 mg, and 400 mg as the free form of the compound I for use in combination with abiraterone acetate to be administered once a day in a dose selected from the group consisting of 250 mg, 500 mg, 750 mg, and 1000 mg in the treatment of castration-resistant prostate cancer with bone metastasis by being administered to a human.

More preferably, the invention relates to the compound I or a pharmaceutically acceptable salt thereof to be administered once a day in a dose selected from the group consisting of 200 mg, 300 mg, and 400 mg as the free form of the compound I for use in combination with abiraterone acetate to be administered once a day in a dose selected from the group consisting of 500 mg, 750 mg, and 1000 mg in the treatment of castration-resistant prostate cancer with bone metastasis by being administered to a human.

More preferably, the invention relates to the compound I or a pharmaceutically acceptable salt thereof to be administered once a day in a dose selected from the group consisting of 200 mg, 300 mg, and 400 mg as the free form of the compound I for use in combination with abiraterone acetate to be administered once a day in a dose selected from the group consisting of 750 mg and 1000 mg in the treatment of castration-resistant prostate cancer with bone metastasis by being administered to a human.

More preferably, the invention relates to the compound I or a pharmaceutically acceptable salt thereof to be administered once a day in a dose selected from the group consisting of 200 mg and 300 mg as the free form of the compound I for use in combination with abiraterone acetate to be administered once a day in a dose selected from the group consisting of 750 mg and 1000 mg in the treatment of castration-resistant prostate cancer with bone metastasis by being administered to a human.

More preferably, the invention relates to the compound I or a pharmaceutically acceptable salt thereof to be administered once a day in a dose selected from the group consisting of 200 mg and 300 mg as the free form of the compound I for use in combination with abiraterone acetate to be administered once a day in an amount of 1000 mg in the treatment of castration-resistant prostate cancer with bone metastasis by being administered to a human.

More preferably, the invention relates to the compound I or a pharmaceutically acceptable salt thereof to be administered once a day in an amount of 300 mg as the free form of the compound I for use in combination with abiraterone acetate to be administered once a day in an amount of 1000 mg in the treatment of castration-resistant prostate cancer with bone metastasis by being administered to a human.

More preferably, the invention relates to the compound I or a pharmaceutically acceptable salt thereof to be administered once a day in an amount of 300 mg as the free form of the compound I for 5 days and then taking a drug holiday for 2 days for use in combination with abiraterone acetate to be administered once a day in an amount of 1000 mg in the treatment of castration-resistant prostate cancer with bone metastasis by being administered to a human.

In addition, the present invention relates to use of the compound I or a pharmaceutically acceptable salt thereof for manufacturing an antitumor agent to be administered in combination with abiraterone or a derivative thereof.

More preferably, the invention relates to use of the compound I or a pharmaceutically acceptable salt thereof for manufacturing an antitumor agent to be administered in combination with abiraterone acetate.

More preferably, the invention relates to use of the compound I or a pharmaceutically acceptable salt thereof for manufacturing an antitumor agent to be administered to a human in combination with abiraterone acetate.

More preferably, the invention relates to use of the compound I or a pharmaceutically acceptable salt thereof for manufacturing a therapeutic agent for castration-resistant prostate cancer to be administered to a human in combination with abiraterone acetate.

More preferably, the invention relates to use of the compound I or a pharmaceutically acceptable salt thereof for manufacturing a therapeutic agent for castration-resistant prostate cancer to be administered in a dose of from 200 mg to 400 mg as the free form of the compound I to a human in combination with abiraterone acetate in a dose of from 250 mg to 1000 mg.

More preferably, the invention relates to use of the compound I or a pharmaceutically acceptable salt thereof for manufacturing a therapeutic agent for castration-resistant prostate cancer with bone metastasis to be administered in a dose of from 200 mg to 400 mg as the free form of the compound I to a human in combination with abiraterone acetate in a dose of from 250 mg to 1000 mg.

More preferably, the invention relates to use of the compound I or a pharmaceutically acceptable salt thereof for manufacturing a therapeutic agent for castration-resistant prostate cancer with bone metastasis to be administered once a day in a dose of from 200 mg to 400 mg as the free form of the compound I to a human in combination with abiraterone acetate to be administered once a day in a dose of from 250 mg to 1000 mg.

More preferably, the invention relates to use of the compound I or a pharmaceutically acceptable salt thereof for manufacturing a therapeutic agent for castration-resistant prostate cancer with bone metastasis to be administered once a day in a dose selected from the group consisting of 200 mg, 300 mg, and 400 mg as the free form of the compound I to a human in combination with abiraterone acetate to be administered once a day in a dose selected from the group consisting of 250 mg, 500 mg, 750 mg, and 1000 mg.

More preferably, the invention relates to use of the compound I or a pharmaceutically acceptable salt thereof for manufacturing a therapeutic agent for castration-resistant prostate cancer with bone metastasis to be administered once a day in a dose selected from the group consisting of 200 mg, 300 mg, and 400 mg as the free form of the compound I to a human in combination with abiraterone acetate to be administered once a day in a dose selected from the group consisting of 500 mg, 750 mg, and 1000 mg.

More preferably, the invention relates to use of the compound I or a pharmaceutically acceptable salt thereof for manufacturing a therapeutic agent for castration-resistant prostate cancer with bone metastasis to be administered once a day in a dose selected from the group consisting of 200 mg, 300 mg, and 400 mg as the free form of the compound I to a human in combination with abiraterone acetate to be administered once a day in a dose selected from the group consisting of 750 mg and 1000 mg.

More preferably, the invention relates to use of the compound I or a pharmaceutically acceptable salt thereof for manufacturing a therapeutic agent for castration-resistant prostate cancer with bone metastasis to be administered once a day in a dose selected from the group consisting of 200 mg and 300 mg as the free form of the compound I to a human in combination with abiraterone acetate to be administered once a day in a dose selected from the group consisting of 750 mg and 1000 mg.

More preferably, the invention relates to use of the compound I or a pharmaceutically acceptable salt thereof for manufacturing a therapeutic agent for castration-resistant prostate cancer with bone metastasis to be administered once a day in a dose selected from the group consisting of 200 mg and 300 mg as the free form of the compound I to a human in combination with abiraterone acetate to be administered once a day in an amount of 1000 mg.

More preferably, the invention relates to use of the compound I or a pharmaceutically acceptable salt thereof for manufacturing a therapeutic agent for castration-resistant prostate cancer with bone metastasis to be administered once a day in an amount of 300 mg as the free form of the compound I to a human in combination with abiraterone acetate to be administered once a day in an amount of 1000 mg.

More preferably, the invention relates to use of the compound I or a pharmaceutically acceptable salt thereof for manufacturing a therapeutic agent for castration-resistant prostate cancer with bone metastasis to be administered to a human once a day in an amount of 300 mg as the free form of the compound I for 5 days and then taking a drug holiday for 2 days in combination with abiraterone acetate to be administered once a day in an amount of 1000 mg.

### Examples

Examples and Reference Examples will be shown below to describe the combinational administration of the compound I or a pharmaceutically acceptable salt thereof and abiraterone or a derivative thereof of the present invention in more detail. The present invention is not limited to these Examples.

### <Reference Example 1: Preparation of methanesulfonate salt of compound I>

Compound I was prepared by the method described in Patent Literature 1, and a methanesulfonate salt of the compound I was prepared by the method described in Patent Literature 5. The preparation can be conducted in accordance with the method described in Patent Literature 6.

### <Reference Example 2: Preparation of abiraterone acetate>

Abiraterone acetate was obtained from commercial product or based on information known in literature.

### <Example: Combinational administration test of methanesulfonate salt of compound I and abiraterone acetate>

A methanesulfonate salt of compound I and abiraterone acetate were administered to patients of castration-resistant prostate cancer with bone metastasis as subjects, and the efficacy and side effects were evaluated. A schedule of administering the methanesulfonate salt of compound I in any of three doses in terms of the free form (200, 300, or 400 mg/body/day) to patients who were receiving secondary endocrine therapy before docetaxel treatment as subjects once a day for 5 days and taking a drug holiday of the compound I only for 2 days was repeated. During this period, 1000 mg of abiraterone acetate was administered to every dosage groups every day once a day, and six subjects were examined in each dosage group. When "side effects of grade 2 or higher continuing without being improved or recovered by symptomatic treatment or side effects of grade 3 or higher (excluding abnormal clinical examination values) " due to the compound I were observed in two or more subjects on the 21st day from the start of administration of the compound I in each dosage group, discontinuation of recording and evaluation, etc. of the dosage group or the group exceeding the dosage were considered by the Steering Committee. In the side effects that determine this discontinuation of enrollment and evaluation, etc., abnormal clinical examination values, such as an increase in alanine aminotransferase (ALT), an increase in aspartate aminotransferase (AST), and an increase in blood creatine kinase (CK), are not included. In addition, after completion of the evaluation of each dosage group, the propriety of increasing the number of cases for evaluating the side effects and efficacy was judged by the Steering Committee, and an increase in the number of cases was allowed within a range of not exceeding 24 as the number of cases for evaluation of the whole present cohort in a tolerable dose. Accordingly, since the number of cases for evaluation of each dosage group is small, i.e., 6 cases, from the viewpoint of the efficacy, side effects, and administration continuity, 6 cases at 300 mg/body/day were additionally evaluated.

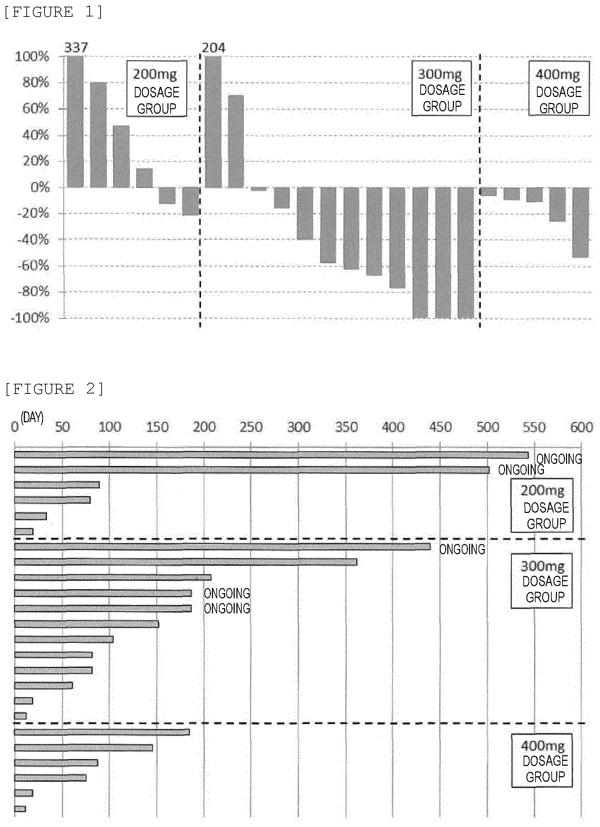

Regarding the efficacy, bone scintigraphy to be used for detecting bone metastasis was implemented, and evaluation was performed based on Bone Scan Index (BSI) using analytical data by BONE NAVI and criteria of Response Evaluation Criteria in Solid Tumors (RECIST) guideline Version 1.1 or Prostate Cancer Clinical Trials Working Group 3 (PCWG3). The BSI is an index for evaluating the spread of bone metastasis and calculates the ratio of the high accumulation area (the Artificial Neural Networks (ANN value) is higher than 0.5) that seems to be a tumor to the bone mass of the whole body. The ANN value indicates the degree of confidence of metastasis on a scale from 0 to 1, and an ANN value exceeding 0.5 is assessed to have a risk of bone metastasis. In addition, in Figure 1, the best value among BSI measured during the period of administration of the compound I is shown as Best response based on the bone metastasis on the day of starting the administration in each dosage group. That is, in Figure 1, 0% indicates that the BSI value did not change during the period of administration of the compound I, and 100% indicates that the high accumulation area where the ANN value exceeded 0.5 increased during the period and that the BSI value was doubled. In contrast, -100% indicates that the high accumulation area where the ANN value exceeded 0.5 disappeared during the period and that the BSI value became 0.

As a result, in 200 mg, suppression of bone metastasis was observed in two of six cases. In 300 mg, suppression of bone metastasis was observed in 10 in 12 cases, and the BSI value became 0 in three cases therein. In 400 mg, bone metastasis could not be measured in one of six cases, and suppression of bone metastasis was observed in five of the remaining five cases.

The grade of the seriousness of side effects was judged using Common Terminology Criteria for Adverse Events (CTCAE) Ver. 4.03. In every group, "side effects of grade 3 or higher continuing without being improved or recovered by symptomatic treatment" were not recognized in two or more subjects.

Furthermore, Figure 2 shows the number of days that the compound I and abiraterone in each dosage group could be administered. That is, a larger number of the administration days allows to expect an antitumor effect and also indicates that the dose is a dose that can be more safely administered. In addition, the statement of "ongoing" indicates that the combination test was ongoing when the specification and so on of the present application were produced, and the average numbers of administration days calculated for the respective dosage groups at that time were 211 days in the 200 mg dosage group, 158 days in the 300 mg dosage group, and 87 days in the 400 mg dosage group. Consequently, in every dosage groups, the average number of administration days exceeded 50 days, in particular, it was demonstrated that the average numbers of administration days of the 200 mg and 300 mg dosage groups exceeded 100 days.

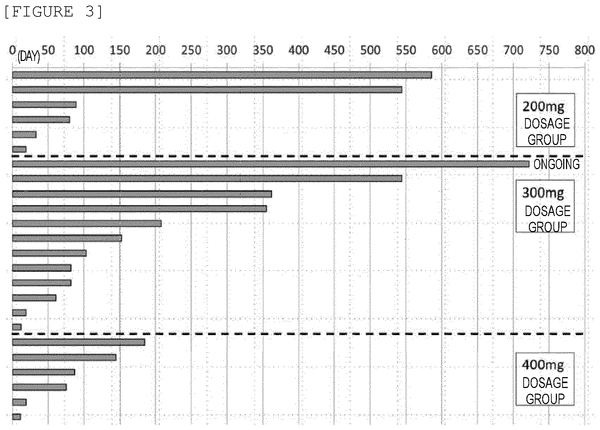

Furthermore, Figure 3 shows the number of days that the compound I and abiraterone in each dosage group could be administered at the time after about one year from the time when the results shown in Figure 2 were obtained. That is, a larger number of the administration days allows to expect an antitumor effect and also indicates that the dose is a dose that can be more safely administered. In addition, the statement of "ongoing" indicates that the combination test was ongoing when the specification and so on of the present application were produced, and the average numbers of administration days calculated for the respective dosage groups at that time were 225 days in the 200 mg dosage group, 225 days in the 300 mg dosage group, and 87 days in the 400 mg dosage group. Consequently, in every dosage groups, the average number of administration days exceeded 50 days, in particular, it was demonstrated that the average numbers of administration days of the 200 mg and 300 mg dosage groups exceeded 200 days.

The results above demonstrate that in each dosage group (200 mg, 300 mg, and 400 mg) of the compound I, suppression of bone metastasis in patients with castration-resistant prostate cancer was observed by administration in combination with an abiraterone acetate ester, in particular, in the 300 mg and 400 mg dosage groups, the frequency of suppression was high. Furthermore, in the 300 mg dosage group, an effect of making the BSI value 0 was also recognized. In addition, in every dosage groups, the side effects were acceptable. It was also demonstrated that it is possible to continue the combinational administration in every dosage groups, it was demonstrated that it is possible to continue the combinational administration, in particular, the results of the average number of administration days in the 200 mg and 300 mg dosage groups demonstrated that the antitumor effect and the safety aspect can be expected.

It was thus demonstrated that combination of the compound I and an abiraterone derivative can exhibit an antitumor effect.

## Claims

1. An antitumor agent comprising a compound represented by the following formula (I) or a pharmaceutically acceptable salt thereof: 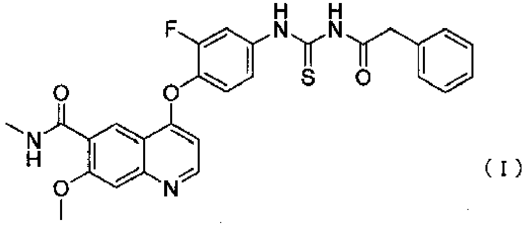 wherein the agent is administered in combination with abiraterone or a derivative thereof.

2. The antitumor agent according to claim 1, wherein the pharmaceutically acceptable salt is a methanesulfonate salt.

3. The antitumor agent according to claim 1 or 2, wherein the abiraterone or a derivative thereof is abiraterone acetate.

4. The antitumor agent according to claims 1 to 3, being administered to a human.

5. The antitumor agent according to any one of claims 1 to 4, for treating castration-resistant prostate cancer.

6. The antitumor agent according to any one of claims 1 to 5, for treating castration-resistant prostate cancer with bone metastasis.

7. The antitumor agent according to any one of claims 1 to 6, wherein the compound represented by formula (I) or a pharmaceutically acceptable salt thereof is administered once a day in an amount of from 200 mg to 400 mg as a free form of the compound I.

8. The antitumor agent according to any one of claims 1 to 6, wherein the compound represented by formula (I) or a pharmaceutically acceptable salt thereof is administered once a day in an amount of 300 mg as a free form of the compound I.

9. The antitumor agent according to any one of claims 1 to 8, wherein the compound represented by formula (I) or a pharmaceutically acceptable salt thereof is administered for 5 days, and a drug holiday for 2 days is then taken.

10. An antitumor effect-enhancing agent for abiraterone or a derivative thereof, comprising a compound represented by the following formula (I) or a pharmaceutically acceptable salt thereof: 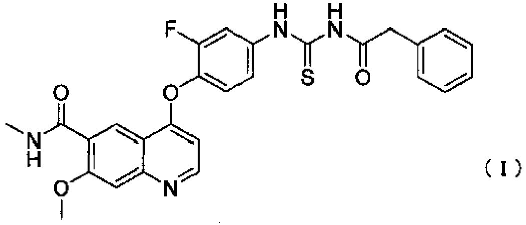 as an active ingredient.

11. A method for treating a tumor, comprising administering therapeutically effective amounts of a compound represented by the following formula (I) or a pharmaceutically acceptable salt thereof: 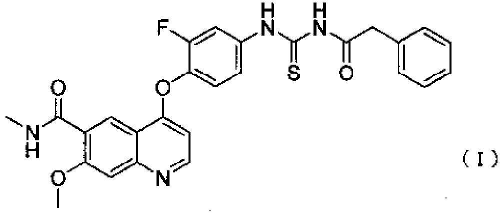 and abiraterone or a derivative thereof to a patient.

12. A compound represented by the following formula (I) or a pharmaceutically acceptable salt thereof: 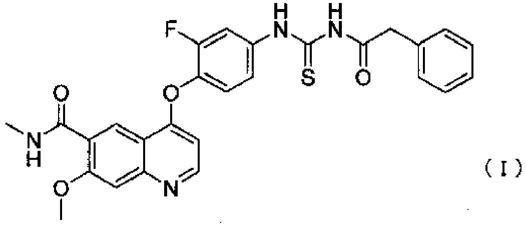 for use in combination with abiraterone or a derivative thereof in the treatment of a tumor.

13. Use of a compound represented by the following formula (I) or a pharmaceutically acceptable salt thereof: 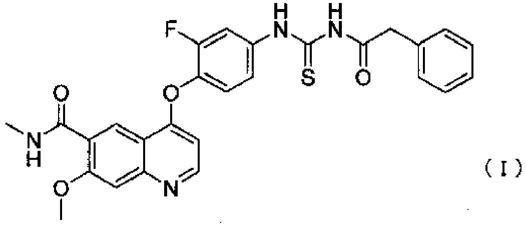 for manufacturing an antitumor agent to be administered in combination with abiraterone or a derivative thereof.

14. A method for enhancing an antitumor effect of abiraterone or a derivative thereof, comprising administering a therapeutically effective amount of a compound represented by the following formula (I) or a pharmaceutically acceptable salt thereof: 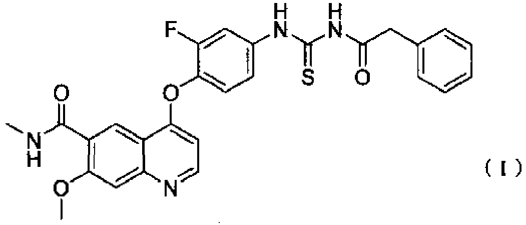 to a patient.

15. A compound represented by the following formula (I) or a pharmaceutically acceptable salt thereof: 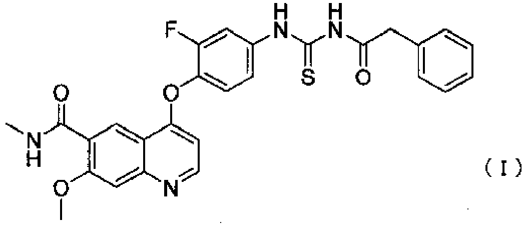 for enhancing an antitumor effect of abiraterone or a derivative thereof.

16. Use of a compound represented by the following formula (I) or a pharmaceutically acceptable salt thereof: 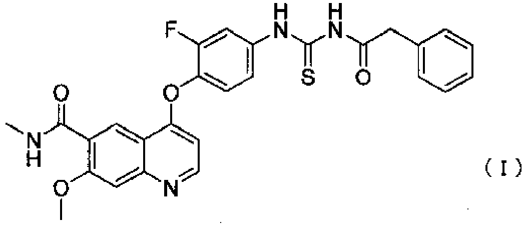 for manufacturing an antitumor effect-enhancing agent for enhancing an antitumor effect of abiraterone or a derivative thereof.
